(19) Europäisches Patentamt
European Patent Office
Office européen des brevets

(11) Veröffentlichungsnummer : **0 481 921 A1**

# (12) EUROPÄISCHE PATENTANMELDUNG

(21) Anmeldenummer : **91810787.1**

(22) Anmeldetag : **09.10.91**

(51) Int. Cl.[5] : **C07C 335/18,** C07C 267/00, A01N 47/30, A01N 47/40

(30) Priorität : **18.10.90 CH 3333/90**

(43) Veröffentlichungstag der Anmeldung : **22.04.92 Patentblatt 92/17**

(84) Benannte Vertragsstaaten : **BE CH DE DK ES FR GB GR IT LI LU NL**

(71) Anmelder : **CIBA-GEIGY AG**
**Klybeckstrasse 141**
**CH-4002 Basel (CH)**

(72) Erfinder : **Ehrenfreund, Josef, Dr.**
**Amselstrasse 11**
**CH-4123 Allschwil (CH)**
Erfinder : **Drabek, Jozef, Dr.**
**Benkenstrasse 12**
**CH4104 Oberwil (CH)**

## (54) Substituierte Carbodiimide und Thioharnstoffe und ihre Verwendung bei der Bekämpfung von Schädlingen.

(57) Neue 3,5-Diisopropyl-diphenyläther-Derivate der Formel I

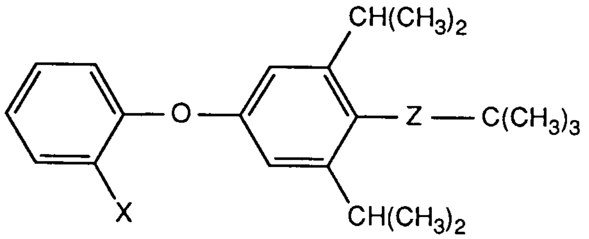

(I)

worin X Fluor oder Chlor und
Z die Brücke -NH-CS-NH- oder -N=C=N- bedeuten, können als Schädlingsbekämpfungsmittel eingesetzt werden. Vorzugsweise werden Insekten und Spinnmilben bekämpft.

EP 0 481 921 A1

Die vorliegende Erfindung betrifft neue Derivate von 3,5-Diisopropyl-diphenyläthern, Verfahren zu ihrer Herstellung, Schädlingsbekämpfungsmittel, welche diese Verbindungen enthalten, sowie ihre Verwendung bei der kontrolle von Schädlingen aus dem kreis der Arthropoda, vorzugsweise von Insekten und Vertretern der Ordnung Akarina.

Die erfindungsgemässen 3,5-Diisopropyl-diphenyläther-Derivate entsprechen der Formel I

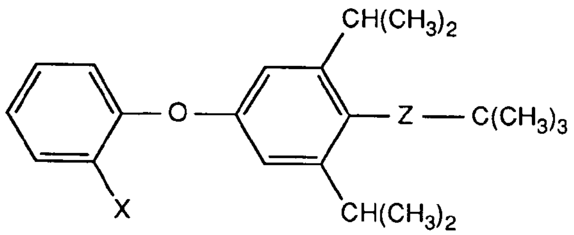

(I)

worin X Fluor oder Chlor und
Z die Brücke -NH-CS-NH- oder -N=C=N- bedeuten.

Aus der Literatur sind insektizid wirkende Phenylthioharnstoffe und Phenylcarbodiimide mit Diphenylätherstruktur aus der DE-OS 3034905 und EP-A-175649 bekannt. Die biologischen Eigenschaften der dort offenbarten Verbindungen vermögen jedoch bei der Schädlingsbekämpfung nicht voll zu befriedigen. Es besteht daher ein Bedürfnis nach Wirkstoffen aus dieser klasse mit verbesserten Eigenschaften zur Bekämpfung von Insekten und Vertretern der Ordnung Akarina. Überraschenderweise wird dieses Bedürfnis von den erfindungsgemässen Wirkstoffen weitgehend befriedigt.

Die Gruppe der Verbindungen der Formel I zerfällt in die der 2,6-Diisopropyl-4-phenoxy-phenyl-thioharnstoffe der Formel Ia

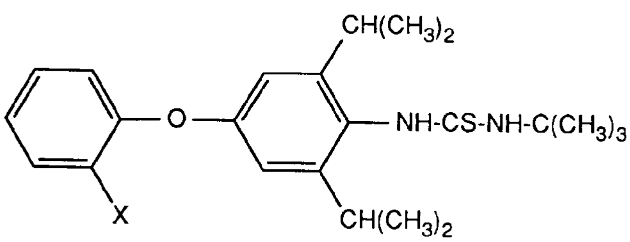

(Ia)

und die der 2,6-Diisopropyl-4-phenoxy-phenyl-carbodiimide der Formel Ib

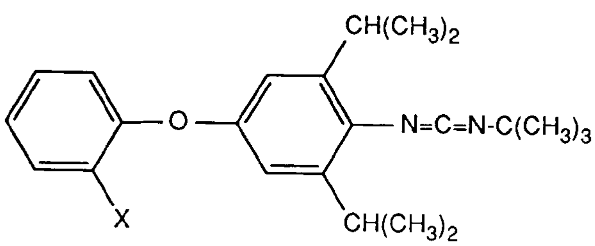

(Ib)

worin X Fluor oder Chlor bedeutet.

Bevorzugt sind jeweils die Wirkstoffe der Formeln Ia und Ib, worin X Chlor bedeutet, also die Verbindungen N-2,6-Diisopropyl-4-(2-chlorphenoxy)-phenyl-N'-tert.butyl-thio-harnstoff und N-2,6-Diisopropyl-4-(2-chlorphenoxy)-phenyl-N'-tert.butyl-carbodiimid.

Die erfindungsgemässen Verbindungen der Formel I können in Analogie zu bekannten Verfahren hergestellt werden. Beispielsweise erhält man die Verbindung der Formel I, indem man

a) ein Isothiocyanat der Formel II

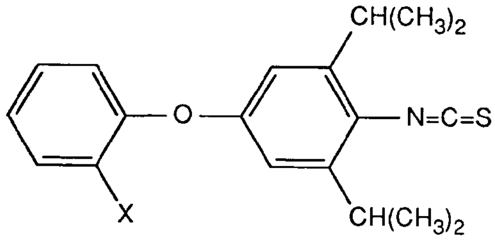

(II)

worin X die unter Formel I gegebene Bedeutung hat, mit tert.Butylamin der Formel III

$$H_2N\text{-}C(CH_3)_3 \qquad (III)$$

zum Thioharnstoff der Formel Ia

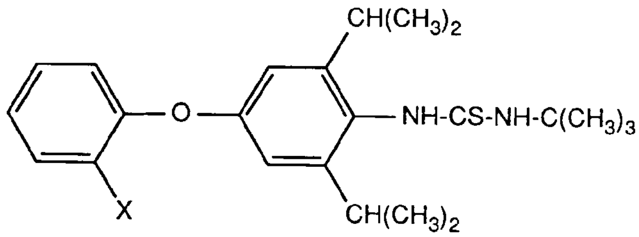

(Ia)

umsetzt und gewünschtenfalls

b) den erhaltenen Thioharnstoff der Formel Ia durch Abspalten von Schwefelwasserstoff in das Carbodiimid der Formel Ib

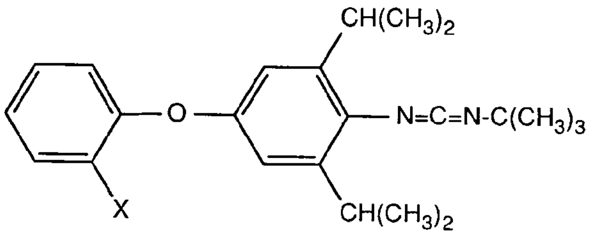

(Ib)

überführt.

Das Verfahren a) wird üblicherweise unter normalem Druck, und in Gegenwart eines organischen Lösungs- oder Verdünnungsmittels durchgeführt. Die Temperatur beträgt dabei zwischen +0°C und +150°C, vorzugsweise +10°C bis +70°C. Als Lösungs- oder Verdünnungsmittel eignen sich beispielsweise Aether und ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon, Methylisobutylketon oder Cyclohexanon.

Das Verfahren b) wird zweckmässigerweise auf übliche Weise in einem aprotischen organischen Lösungs- oder Verdünnungsmittel unter normalem Druck durchgeführt. Die Temperatur beträgt dabei zwischen +0°C und +150°C, vorzugsweise +10°C bis +50°C.

Geeignete Lösungs- oder Verdünnungsmittel sind zum Beispiel Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; ketone, wie Aceton, Methyläthylketon, Methylisopropylketon oder Cyclohexanon. Die Abspaltung von Schwefelwasserstoff erfolgt dabei nach in der Literatur beschriebenen Arbeitsweisen (Chemistry Letters 1977, p. 575-76; Tetrahedron Letters 1985, p. 1661-64; Ber. Dtsch. Chem. Ges. 6, 1873, p. 1398; Bull. Soc. Chim. 1956, p. 1360). Als Abspaltungs-Reagenzien werden dabei unter anderem HgO, bestimmte Pyridinium Salze, Chloressigsäureester, Cyanursäurechlorid, p-Toluolsulfonsäurechlorid oder bestimmte Phosphorsäureester-Derivate verwendet.

Das Verfahren b) kann neben der skizzierten Verfahrensweise auch auf photochemischem Wege erfolgen (EP-A-307361), indem man den Thioharnstoff der Formel Ia photochemisch zum Carbodiimid der Formel Ib mit Sauerstoff oxidiert.

Die Photooxidation kann beispielsweise mit Licht mit einer Wellenlänge von bevorzugt 200 bis 700 nm vorgenommen werden. Bei der Verwendung von UV-Licht zum Beispiel im UV-B-Bereich kann auf die Mitverwendung von Sensibilisatoren verzichtet werden. Es hat sich als zweckmässig erwiesen, dass man die Photooxidation mit UV-Licht oder sichtbarem Licht in Gegenwart eines Sensibilisators durchführt. Geeignete Lichtquellen sind Sonnenlicht, Halogenlampen, Glühlampen für Belichten von aussen, Natriumdampflampen oder Quecksilberdampflampen (als UV-Lichtquelle). Geeignete Sensibilisatoren zur Erzeugung von Singlettsauerstoff sind z.B.: Xanthenfarbstoffe (Bengalrosa), Thiazine (Methylenblau), Porphyrine (Tetraphenylporphyrin), Thionin, Eosin, Erythrosin, Phenosafranin, Chlorophyll, Flavine, Thioxanthone, Phthalocyanine, Thiophene, Naphthalinderivate, Phenothiazine, Pyrazolanthrone, Ketocumarine, Azine (Riboflavin), Anthrachinone, Metallozene, Benzophenone, Anthracenderivate. Eine bevorzugte Gruppe ist Methylenblau, Bengalrosa, Tetraphenylporphyrin und Phthalocyanine. Die Reaktion kann bei einer Temperatur von z.B. -20°C bis 50°C durchgeführt werden, bevorzugt bei Raumtemperatur (etwa 15°C bis 35°C). Als Lösungsmittel eignen sich inerte organische Lösungsmittel und Lösungsmittelgemische und deren Mischungen mit Wasser. Geeignete Lösungsmittel sind beispielsweise aliphatische, cycloaliphatische und aromatische Kohlenwasserstoffe (Pentan, Hexan, Cyclohexan, Benzol, Toluol), Chlorkohlenwasserstoffe (Methylenchlorid, Chloroform, Tetrachlorkohlenstoff, Tri- oder Tetrachloräthan, Chlorbenzol), Alkohole (Methanol, Aethanol, Aethylenglykolmonomethyläther), Aether (Diäthyläther , Dibutyläther, Aethylenglykoldiäthyläther, Tetrahydrofuran, Dioxan), ketone (Methylisobutylketon), Ester (Essigsäureäthylester), Nitrile (Acetonitril), N,N-disubstituierte Carbonsäureamide und Lactame (Dimethylacetamid, N-Methylpyrrolidon), Sulfone (Tetramethylensulfon). Ein bevorzugtes Lösungsmittel ist ein Gemisch aus Acetonitril und Wasser. Bei der Reaktion entsteht Schwefelsäure. Es werden daher zweckmässig mindestens 2 Aequivalente, beispielsweise 2 bis 2,5 Aequivalente eines Säurefängers verwendet. Bei dem Säurefänger kann es sich zum Beispiel um eine Alkali- oder Erdalkalimetallbase, ein Alkalicarbonat oder -hydrogencarbonat oder eine Pufferlösung mit einem pH $\geqq$ 7 handeln. Beispiele sind LiOH, KOH, NaOH, $Ca(OH)_2$, $NaHCO_3$, $CaHCO_3$, $kHCO_3$. Geeignete Puffermischungen mit pH $\geqq$ 7 sind unter anderem wässrige Lösungen von Borax/NaCl oder $KH_2PO_4$, $K_3PO_4$, Borax, $NaHCO_3$, $Na_2HPO_4$ oder kCl und NaOH. Bevorzugt wird NaOH verwendet. Das Verfahren kann so durchgeführt werden, dass man gasförmigen Sauerstoff, zum Beispiel reinen Sauerstoff, Luft oder Sauerstoff im Gemisch mit Inertgasen in das Reaktionsgemisch leitet. Inertgase sind beispielsweise Stickstoff, kohlendioxid und Edelgase, wie Helium, Neon und Argon. Bevorzugt ist die Verwendung von Singlettsauerstoff, besonders wenn Sensibilisatoren mitverwendet werden. Das photochemische Verfahren b) kann im einzelnen so durchgeführt werden, dass man die Verbindung der Formel Ia, den Säurefänger, das Lösungsmittel und gegebenenfalls den Sensibilisator vorlegt und das Gemisch unter Belichtung im offenen System in Gegenwart von Luft gut rührt oder unter Rühren und Belichten Sauerstoff oder ein Sauerstoff/Inertgas-Gemisch durch die Reaktionsmischung leitet. Die Aufarbeitung des Reaktionsgemisches erfolgt in üblicher Weise durch Extraktion, Waschen und Trocknen des Extraktes und Abdestillieren des Lösungsmittels.

Die Isothiocyanate der Formel II können nach im Prinzip bekannten Methoden hergestellt werden, indem man ein Anilin der Formel IV

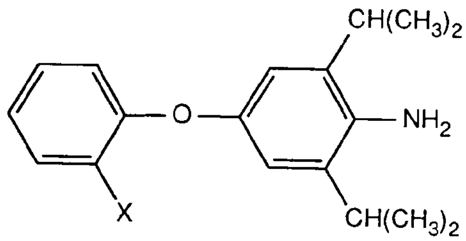

(IV)

worin X die unter Formel I angegebene Bedeutung hat, mit Thiophosgen umsetzt.

Das Verfahren zur Herstellung der Verbindungen der Formel II wird zweckmässigerweise in Gegenwart einer organischen oder anorganischen Base, wie beispielsweise Triäthylamin oder Calciumcarbonat, und einem gegenüber den Reaktionsteilnehmern inerten Lösungs- oder Verdünnungsmittels unter normalem Druck durchgeführt. Die Temperatur beträgt dabei zwischen 0°C und +100°C, vorzugsweise Siedetemperatur des verwendeten Lösungs- oder Verdünnungsmittels oder +20°C bis +80°C. Geeignete Lösungs- oder Verdünnungsmittel sind unter anderem Aether oder ätherartige Verbindungen, wie Diäthyläther, Diisopropyläther, Dioxan oder Tetrahydrofuran; aromatische kohlenwasserstoffe, wie Benzol, Toluol oder Xylole; Ketone, wie Aceton, Methyläthylketon oder Cyclohexanon; oder chlorierte Kohlenwasserstoffe wie Dichlormethan oder Tetrachlorkohlenstoff. Die Herstellung kann auch in Anwesenheit von Wasser im 2-Phasensystem erfolgen.

Die Phenoxyaniline der Formel IV können nach im Prinzip bekannten Methoden beispielsweise hergestellt werden, indem man ein Anilin der Formel V

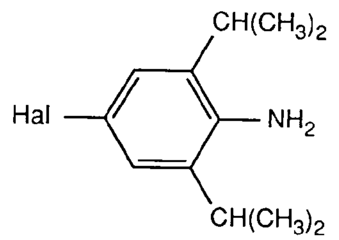

(V)

mit einem Phenol der Formel VI

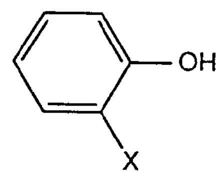

(VI)

umsetzt, wobei X die unter Formel I angegebene Bedeutung hat und Hal für Halogen, insbesondere für Chlor und Brom steht.

Das Verfahren zur Herstellung der Verbindungen der Formel IV wird zweckmässigerweise in Gegenwart einer organischen oder insbesondere anorganischen Base, wie beispielsweise einem Alkalihydroxid oder -carbonat, und einem gegenüber den Reaktionsteilnehmern inerten, vorzugsweise polaren Lösungs- oder Verdünnungsmittels unter normalem Druck durchgeführt. Die Temperatur beträgt dabei 0 bis +200°C, vorzugsweise Siedetemperatur des verwendeten Lösungs- oder Verdünnungsmittels oder +50 bis +170°C. Als vorteilhaft kann sich auch der Zusatz eines Schwermetall-katalysators wie z.B. kupferpulver oder basisches kupfer-II-carbonat erweisen. Geeignete Lösungs- und Verdünnungsmittel sind u.a. Amide wie Dimethylformamid, Dimethylsulfoxid, N-Methylpyrrolidon und andere aprotisch dipolare Lösungsmittel.

Die Verbindungen der Formeln II und IV sind neu und bilden ebenfalls einen Gegenstand der vorliegenden Erfindung. Die Verbindungen der Formeln III, V und VI sind dagegen bekannt und zum Teil im Handel erhältlich oder können nach im Prinzip bekannten Methoden hergestellt werden.

Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:

aus der Ordnung Lepidoptera zum Beispiel

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp.,

Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung der Orthoptera zum Beispiel
Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.;
aus der Ordnung der Psocoptera zum Beispiel
Liposcelis spp.;
aus der Ordnung der Anoplura zum Beispiel
Haematopinus spp., Linognathus spp. Pediculus spp., Pemphigus spp. und Phylloxera spp.; aus der Ordnung der Mallophaga zum Beispiel
Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera zum Beispiel
Ceratophyllus spp., Xenopsylla cheopis,
aus der Ordnung der Acarina zum Beispiel
Acarus siro, Aceria sheldoni, Aculus schlechtendali, Amblyomma spp., Argas spp.,
Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Die Verbindungen eignen sich besonders zur Bekämpfung von Schädlingen in Baumwolle-, Obst-, Citrus- und Gemüsekulturen. Insbesondere werden Spinnmilben wie Tetranychus urticae, Panonychus ulmi, Panonychus citri, Phyllocoptruta oleivora, Tetranychus cinnabarinus, fressende Insektenlarven wie die von Plutella xylostella und saugende Insekten wie Bemisia tabaci bekämpft.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische

Phosphorverbindungen, Nitrophenole und Derivate, Formamidine, Harnstoffe, Carbamate, Pyrethroide, chlorierte kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu emulgierbaren konzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen der Formel I auch für den Einsatz bei der Behandlung von Saatgut. Dabei kann sowohl das Saatgut vor dem Säen mit dem Wirkstoff oder einer den Wirkstoff enthaltenden Formulierung behandelt oder gebeizt werden, als auch der Wirkstoff beim Säen in die Saatfurche appliziert werden.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$ von Alkylbenzolen wie Xylolgemische oder alkylierteNaphthaline, aliphatische oder cycloaliphatische Kohlenwasserstoffe, wie Cyclohexan, Paraffine oder Tetrahydronaphthalin, Alkohole wie Aethanol, Propanol oder Butanol, und Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetanolalkohol, starke polare Lösungsmittel, wie N-Methyl-2-pyrrolidon, Dimethylsulfoxid oder Dimethylformamid oder Wasser, Pflanzenöle, wie Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin- salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen.

Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen

enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:

"Mc Cutcheon's Detergents and Emulsifiers Annual", Mc Publishing Corp., Glen Rock, NJ, USA, 1988",
H. Stache, "Tensid-Taschenbuch", 2. Aufl., C. Hanser Verlag München, Wien 1981.

M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 % Wirkstoff der Formel I oder kombinationen dieses Wirkstoffs mit andern Insektiziden oder Akariziden, 1 bis 99,9 % eines festen oder Müssigen Zusatzstoffes und 0 bis 25 %, insbesondere 0,1 bis 25 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen (% = Gewichtsprozent)

Emulgierbare Konzentrate:

| | |
|---|---|
| Aktiver Wirkstoff: | 1 bis 90 %, bevorzugt 5 bis 20 % |
| oberflächenaktives Mittel: | 1 bis 30 %, vorzugsweise 10 bis 20 % |
| flüssiges Trägermittel: | 5 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspension-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, z.B. gegebenenfalls epoxidierte Pflanzenöle (epoxidiertes kokosnussöl, Rapsöl oder Sojaöl), Entschäumer, z.B. Silikonöl, konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Herstellungsbeispiele

Beispiel H1: N-2,6-Diisopropyl-4-(2-chlorphenoxy)-phenyl-N'-tert.butyl-thioharnstoff

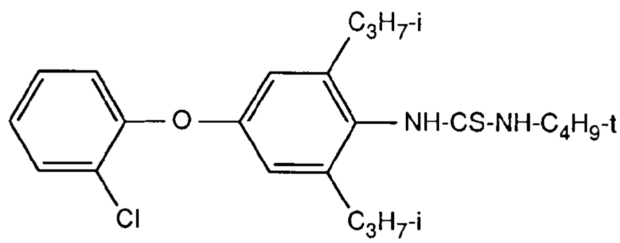

8,5 g 2,6-Diisopropyl-4-(2-chlorphenoxy)-phenylisothiocyanat werden mit 50 ml Tetrahydrofuran verdünnt und anschliessend bei Raumtemperatur mit 3,6 g tert.Butylamin versetzt. Man lässt die Mischung für 24 Stunden bei Raumtemperatur stehen und erhitzt anschliessend noch für 6 Stunden auf +40°C. Das Reaktionsgemisch wird auf Eiswasser gegossen, der entstandene Niederschlag abfiltriert, mit Wasser gewaschen und nach dem Trocknen aus Hexan umkristallisiert. Man erhält so in Form farbloser Kristalle den reinen N-2,6-Diisopropyl-4-(2-chlorphenoxy)-phenyl-N'-tert.butyl-thioharnstoff,
Smp. 151 - 152°C.

Beispiel H2: N-2,6-Diisopropyl-4-(2-fluorphenoxy)-phenyl-N'-tert.butyl-thioharnstoff

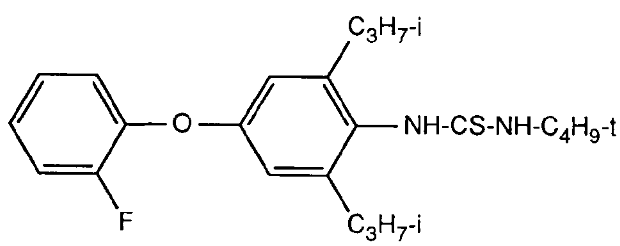

In analoger Verfahrensweise wie in Beispiel H1 erhält man aus 2,6-Diisopropyl-4-(2-fluorphenoxy)-phenylisothiocyanat und tert.Butylamin reinen N-2,6-Diisopropyl-4-(2-fluorphenoxy)-phenyl-N'-tert.butyl-thioharnstoff, Smp. 150- 152°C.

Beispiel H3: N-2,6-Diisopropyl-4-(2-chlorphenoxy)-phenyl-N'-tert.butyl-carbodiimid

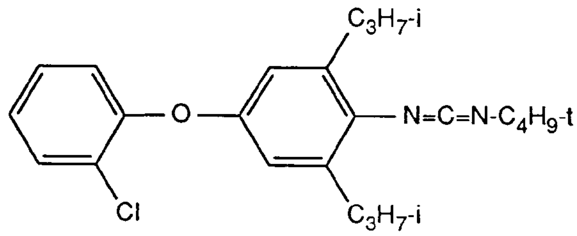

4,0 g gemäss Beispiel H1 erhaltener N-2,6-Diisopropyl-4-(2-chlorphenoxy)-phenyl-N'-tert.butyl-thioharnstoff und 2,9 g 2-Chlor-1-methylpyridiniumjodid werden in 30 ml trockenem Acetonitril vorgelegt, bei Raumtemperatur tropfenweise mit 2,3 g Triäthylamin in 20 ml Acetonitril versetzt und für 30 Minuten bei Siedetemperatur gerührt. Anschliessend wird das Lösungsmittel am Vakuum entfernt, der Rückstand in Hexan aufgenommen und filtriert. Das Filtrat wird dreimal mit Wasser gewaschen, über Natriumsulfat getrocknet, mit kieselgel entfärbt und am Vakuum vom Lösungsmittel befreit. Man erhält so reines N-2,6-Diisopropyl-4-(2-chlorphenoxy)-phenyl-N'-tert.butyl-carbodiimid in Form eines farblosen Oeles, $n_D^{20}$ = 1,5630.

Beispiel H4: N-2,6-Diisopropyl-4-(2-fluorphenoxy)-phenyl-N'-tert.butyl-carbodiimid

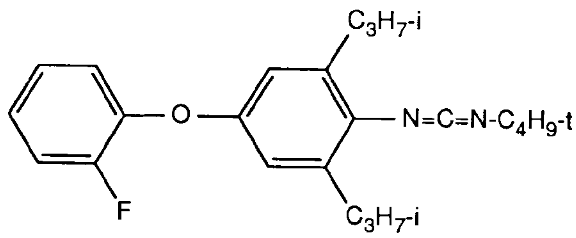

In analoger Verfahrensweise wie in Beispiel H3 erhält man aus 2,6-Diisopropyl-4-(2-fluorphenoxy)-phenyl-N'-tert.butyl-thioharnstoff reines N'-2,6-Diisopropyl-4-(2-fluorphenoxy)-phenyl-N'-tert.butyl-carbodiimid in Form eines farblosen Oeles, $n_D^{23}$ =1,5478.

Beispiel H5: 2,6-Diisopropyl-4-(2-chlorphenoxy)-anilin

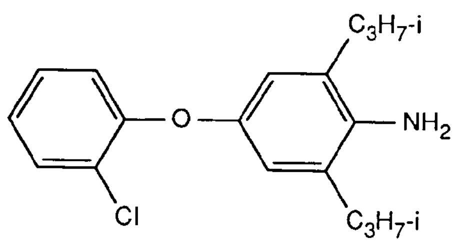

67,2 g 2-Chlorphenol und 7,2 g kaliumcarbonat werden in 200 ml Toluol vorgelegt. Bei Raumtemperatur lässt man innerhalb von 50 Minuten 58,7 g einer 50%igen Kaliumhydroxidlösung zutropfen. Anschliessend wird das entstandene Wasser bei +180°C Badtemperatur azotrop entfernt und nach dem Abkühlen 5,9 g Kupfercarbonat und 350 ml Dimethylformamid zugesetzt. Das Toluol wird abdestilliert bis die Innentemperatur der Mischung +140°C erreicht hat. Bei dieser Temperatur werden 67 g 4-Brom-2,6-diisopropylanilin zugetropft und die Reaktionslösung 20 Stunden gerührt. Das Reaktionsgut wird anschliessend zur Entfernung des Lösungsmittels vollständig eingedampft, mit Diäthyläther verrührt und über Diatomeenerde filtriert. Das Filtrat wird zweimal mit 10%iger Natronlauge, dann mit Wasser gewaschen und über Natriumsulfat getrocknet. Nach Eindampfen der Lösung wird der Rückstand über Kieselgel chromatographiert (Laufmittel: Toluol). Man erhält so 2,6-Diisopropyl-4-(2-chlorphenoxy)-anilin als farbloses Oel, $n_D^{21}$ = 1,5780.

Beispiel H6: 2,6-Diisopropyl-4-(2-fluorphenoxy)-anilin

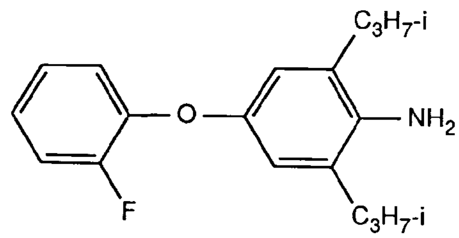

In analoger Verfahrensweise wie im Beispiel H5 erhält man aus 2-Fluorphenol und 4-Brom-2,6-diisopropylanilin reines 2,6-Diisopropyl-4-(2-fluorphenoxy)-anilin in Form farbloser Kristalle, Smp. 82-85°C.

Beispiel H7: 2,6-Diisopropyl-4-(2-chlorphenoxy)-phenylisothiocyanat

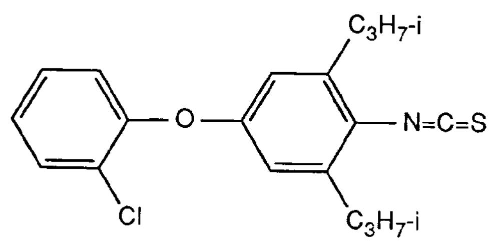

Eine Lösung von 25 g 2,6-Diisopropyl-4-(2-chlorphenoxy)-anilin in 125 ml Dichlormethan werden unter starkem Rühren tropfenweise zu einem Gemisch aus 11,4 g Thiophosgen, 120 ml Dichlormethan, 60 ml Wasser und 18,1 g gemahlenem Calciumcarbonat gegeben. Das Reaktionsgemisch wird für 1 Stunde bei der Siedetemperatur gerührt, dann abgekühlt und über kieselgel filtriert. Die organische Phase wird abgetrennt, mit Wasser gewaschen, über Natriumsulfat getrocknet und im Vakuum vom Lösungsmittel befreit. Man erhält so reines 2,6-Diisopropyl-4-(2′-chlorphenoxy)-phenylisothiocyanat in Form eines gelben Oeles, $n_D^{20}$ =1,6130.

Beispiel H8: 2,6-Diisopropyl-4-(2-fluorphenoxy)-phenylisothiocyanat

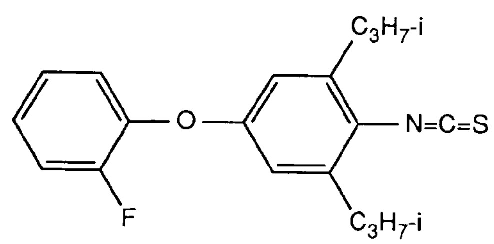

In analoger Verfahrensweise wie im Beispiel H7 erhält man aus 2,6-Diisopropyl-4-(2-fluorphenoxy)-anilin reines 2,6-Diisopropyl-4-(2-fluorphenoxy)-phenylisothiocyanat in Form eines gelben Oeles, $n_D^{23}$ = 1,5979.

Formulierungsbeispiele (% = Gewichtsprozent)

Beispiel F1: Emulsions-Konzentrate

| | a) | b) | c) |
|---|---|---|---|
| Wirkstoff H3 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen konzentraten können durch Verdünnen mit Wasser Emulsionen jeder gewünschten konzentration hergestellt werden.

Beispiel F2: Lösungen

| | a) | b) | c) | d) |
|---|---|---|---|---|
| Wirkstoff H3 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

Beispiel F3: Granulate

| | a) | b) | c) | d) |
|---|---|---|---|---|
| Wirkstoff H4 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschliessend im Vakuum abgedampft.

| Beispiel F4: Stäubemittel | a) | b) |
|---|---|---|
| Wirkstoff H3 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff H1 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Beispiel F6: Emulsions-Konzentrat

| Beispiel F6: Emulsions-Konzentrat | |
|---|---|
| Wirkstoff H1 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

Beispiel F7: Stäubemittel

| | a) | b) |
|---|---|---|
| Wirkstoff H2 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F8: Extruder-Granulat

| | |
|---|---|
| Wirkstoff H1 | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

Beispiel F9: Umhüllungs-Granulat

| | |
|---|---|
| Wirkstoff H2 | 3 % |
| Polyäthylenglykol (MG 200) | 3 % |
| kaolin | 94 % |

Der fein gemahlene Wirkstoff oder die Wirkstoffkombination wird in einem Mischer auf das mit Polyäthylenglykol angefeüchtete Kaolin gleichmässig aufgetragen. Auf diese Weise erhält man staubfreie Umhüllungs-Granulate.

Beispiel F10: Suspensions-Konzentrat

| | |
|---|---|
| Wirkstoff H1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| Silikonöl in Form einer 75 %igen wässrigen Emulsion | 1 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten konzentration hergestellt werden können.

Biologische Beispiele

Beispiel B1: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Formel I zeigen eine gute Wirkung gegen Nilaparvata lugens in diesem Test. Insbesondere die Verbindungen H2, H3 und H4 zeigen eine Wirkung über 80 %.

Beispiel B2: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Formel I zeigen eine gute Wirkung gegen Nephotettix cincticeps in diesem Test. Insbesondere die Verbindungen H2 und H4 zeigen eine Wirkung über 80 %.

Beispiel B3: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.

Die Verbindungen der Formel I zeigen eine gute Wirkung gegen Diabrotica balteata in diesem Test. Insbesondere die Verbindungen H1 und H2 zeigen eine Wirkung über 80 %.

Beispiel B4: Wirkung gegen Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Die Verbindungen der Formel I zeigen gegen Heliothis virescens in diesem Test eine Wirkung über 80 %.

Beispiel B5: Wirkung gegen Spodoptera littoralis Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des dritten Stadiums von Spodoptera littoralis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Die Verbindungen der Formel I zeigen gegen Spodoptera littoralis in diesem Test eine Wirkung über 80 %. Die Verbindungen H2 und H4 reduzieren die Testpopulationen auch noch bei einer Konzentration von 50 ppm um mehr als 80 %.

Beispiel B6: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen der Formel I zeigen eine gute Wirkung gegen Aphis craccivora in diesem Test. Insbesondere die Verbindungen H1, H2 und H4 zeigen eine Wirkung über 80 %.

Beispiel B7: Systemische Wirkung gegen Nilaparvata lupens

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Die Verbindungen der Formel I zeigen eine gute Wirkung gegen Nilaparvata lugens in diesem Test. Insbesondere die Verbindungen H1, H2 und H4 zeigen eine Wirkung über 80 %.

Beispiel B8: Wirkung gegen Boophilus microplus

Vollgesogene adulte Zecken Weibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält, übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Die Verbindungen der Formel I zeigen in diesem Test gegen Boophilus microplus eine Wirkung über 80 %.

Beispiel B9: Wirkung gegen Crocidolomia binotalis Raupen

Junge kohlpflanzen werden mit einer wässrigen Emulsions-Spritzbrühe, die 400 ppm des Wirkstoffes enthält, besprüht. Nach dem Antrocknen des Spritzbelages werden die kohlpflanzen mit 10 Raupen des dritten Stadiums von Crocidolomia binotalis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Die Verbindungen der Formel I zeigen gegen Crocidolomia binotalis in diesem Test eine Wirkung über 80 %.

Beispiel B10: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten von Anthonomus grandis besiedelt und in einen Plastikbehälter gegeben. 3 Tage später erfolgt die Auswertung. Aus dem Vergleich der Anzahl toter käfer und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Die Verbindungen der Formel I zeigen eine gute Wirkung gegen Anthonomus grandis in diesem Test. Insbesondere die Verbindungen H1 und H2 zeigen eine Wirkung über 80 %.

Beispiel B11: Wirkung gegen Bemisia tabaci

Buschbohnen-Pflanzen werden in Gazekäfige gestellt und mit Adulten Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (konzentration 1000 ppm) behandelt. Die Auswertung erfolgt 14 Tage nach der Wirkstoff-Applikation auf %-Schlupf im Vergleich zu den unbehandelten kontrollansätzen.

Die Verbindungen gemäss Formel I zeigen in diesem Test gegen Bemisia tabaci eine Wirkung über 80 %.

Beispiel B12: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm des Wirkstoffes enthält, besprüht. Die Pflanzen werden anschliessend für 6 Tage bei 25°C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.

Die Verbindungen der Formel I zeigen gegen Tetranychus urticae in diesem Test eine Wirkung über 80

%.

Beispiel B13: Wirkung gegen Panonychus ulmi (OP und Carb. resistent)

Apfelsämlinge werden mit adulten Weibchen von Panonychus ulmi besiedelt. Nach 7 Tagen werden die infestierten Pflanzen mit einer wässrigen Emulsion enthaltend 400 ppm der zu prüfenden Verbindung bis zur Tropfennässe besprüht und im Gewächshaus kultiviert. Nach 14 Tagen erfolgt die Auswertung. Aus dem Vergleich der Anzahl toter Spinnmilben auf den behandelten zu denjenigen auf den unbehandelten Pflanzen wird die prozentuale Reduktion an Population (% Wirkung) bestimmt.

Die Verbindungen der Formel I zeigen gegen Panonychus ulmi in diesem Test eine Wirkung über 80 %.

Beispiel 14: Dauerwirkung gegen Tetranychus cinnabarinus

Eingetopfte Bohnenpflanzen im 2-Blattstadium (Sorte "Autan") werden mit wässrigen Wirkstoffemulsionen in den konzentrationen 100 und 200 ppm besprüht. Die applizierten Spritzbrühen werden durch Verdünnen mit Wasser aus 25%igen Emulsionskonzentraten erhalten. Die behandelten Pflanzen werden nach Trocknung des Spritzbelages jeweils nach 2, 4, 8 oder 16 Tagen mit einer gemischten Population des Schädlings Tetranychus cinnabarinus besiedelt. Die infizierten Testpflanzen werden im Gewächshaus bei +26°C, 60 % relativer Luftfeuchtigkeit und einer Beleuchtungsdauer von 14 Stunden pro Tag gehalten. Jeweils 9 Tage nach der Infektion mit dem Schädling wird die Mortalitätswirkung in Prozent ausgewertet.

Die Verbindungen der Formel I zeigen gegen Tetranychus cinnabarinus in diesem Test eine Wirkung über 80 %.

Beispiel B15: Wirkung gegen Dermanyssus gallinae

In einem nach oben offenen Glasbehälter werden 2 bis 3 ml einer 100 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben ermittelt.

Die Verbindungen der Formel I zeigen gute Wirkung gegen Dermanyssus gallinae in diesem Test. Insbesondere die Verbindungen H3 und H4 zeigen eine Wirkung über 80 %.

Beispiel 16: Wirkung gegen Musca domestica

Ein Zuckerwürfel wird mit einer Lösung der Testsubstanz so behandelt, dass die konzentration von Testsubstanz, nach Trocknen über Nacht, im Zucker 250 ppm beträgt. Dieser behandelte Würfel wird mit einem nassen Wattebausch und 10 adulten (ca. 1 Woche alten) Fliegen [Spezies M. domestica; Stamm Schmidlin (Organophosphat-resistent] auf eine Aluminiumschale gelegt. Dieser Versuchsaufbau wird mit einem Becherglas abgedeckt und für 24 Stunden bei 25°C und 50 % Luftfeuchtigkeit belassen. Nach Ablauf dieser Frist wird die Mortalitätsrate bestimmt.

Die Verbindungen der Formel I zeigen eine gute Wirkung im obigen Test. Insbesondere die Verbindung H3 zeigt eine Wirkung über 80 %.

Beispiel B17: Vergleichsversuche mit bekannten Substanzen

Aus dem Stand der Technik (DE-OS 3034905 und EP-A-175649) sind die folgenden Wirkstoffe bekannt:

Verbindung A:

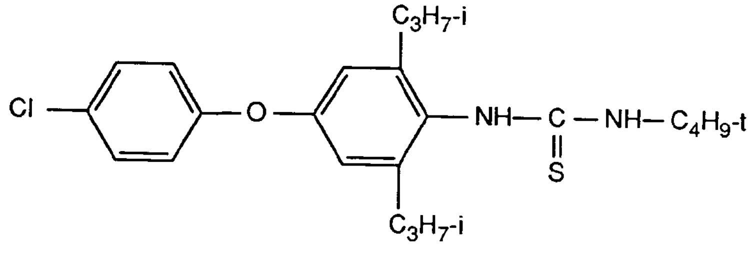

N-2,6-Diisopropyl-4-(4-chlorphenoxy)-phenyl-N'-tert.butyl-thioharnstoff,

Verbindung B:

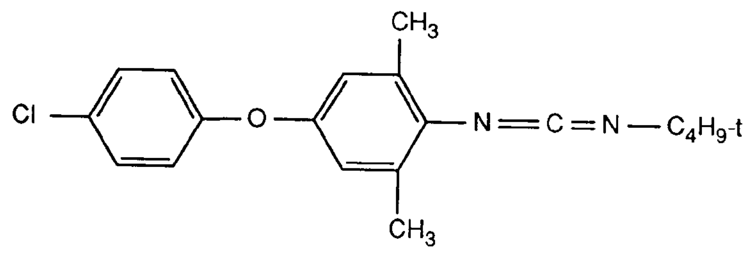

N-2,6-Dimethyl-4-(4-chlorphenoxy)-phenyl-N'-tert.butyl-carbodiimid, und

Verbindung C:

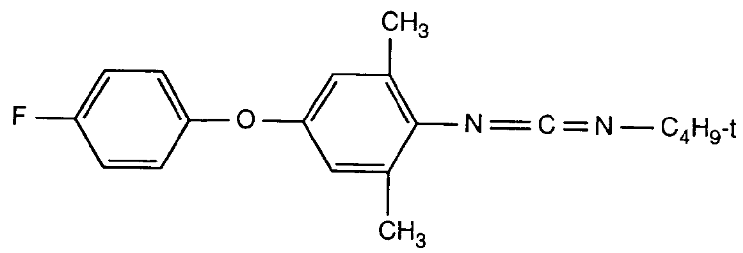

N-2,6-Dimethyl-4-(4-chlorphenoxy)-phenyl-N'-tert.butyl-carbodiimid.

Diese Substanzen werden im Vergleich zu den Wirkstoffen H1, H2, H3 und H4 in den Versuchsaufbauten der Beispiele B4, B5 und B9 geprüft.

Resultate der Vergleiche

| Testorganismus Anwendungskonzentration | Verbindung A | Verbindung H1 | Verbindung H2 |
|---|---|---|---|
| Heliothis (B4) | | | |
| 400 ppm | 35 | 100 | 100 |
| 200 ppm | 0 | 100 | 85 |
| 100 ppm | 0 | 80 | 80 |
| Spodoptera (B5) | | | |
| 400 ppm | 30 | 100 | 100 |
| 200 ppm | 0 | 85 | 100 |
| 100 ppm | 0 | 75 | 100 |
| Crocidolomia (B9) | | | |
| 400 ppm | 0 | 100 | 100 |
| 200 ppm | 0 | 50 | 75 |

| Testorganismus Anwendungs-konzentration | Verbindung B | Verbindung H3 | Verbindung C | Verbindung H4 |
|---|---|---|---|---|
| Heliothis (B4) | | | | |
| 400 ppm | 20 | 100 | 25 | 95 |
| 200 ppm | 0 | 100 | 0 | 70 |
| 100 ppm | 0 | 90 | 0 | 40 |
| Spodoptera (B5) | | | | |
| 400 ppm | 0 | 100 | 0 | 100 |
| 200 ppm | 0 | 100 | 0 | 75 |
| 100 ppm | 0 | 100 | 0 | 55 |
| Crocidolomia (B9) | | | | |
| 400 ppm | 0 | 100 | 0 | 100 |
| 200 ppm | 0 | 100 | 0 | 55 |

## Patentansprüche

1. 3,5-Diisopropyl-diphenyläther-Derivate der Formel I

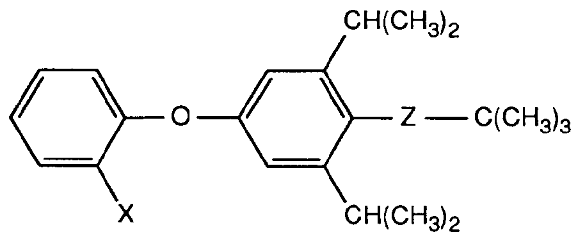

(I)

worin X Fluor oder Chlor und
Z die Brücke -NH-CS-NH- oder -N=C=N- bedeuten.

**2.** Verbindungen gemäss Anspruch 1, dadurch gekennzeichnet, dass X für Chlor steht.

**3.** N-2,6-Diisopropyl-4-(2-chlorphenoxy)-phenyl-N'-tert.butyl-thioharnstoff gemäss Anspruch 1.

**4.** N-2,6-Diisopropyl-4-(2-chlorphenoxy)-phenyl-N'-tert.butyl-carbodiimid gemäss Anspruch 1.

**5.** Verfahren zur Herstellung der Verbindungen der Formel I gemäss Anspruch 1, dadurch gekennzeichnet, dass man
a) ein Isothiocyanat der Formel II

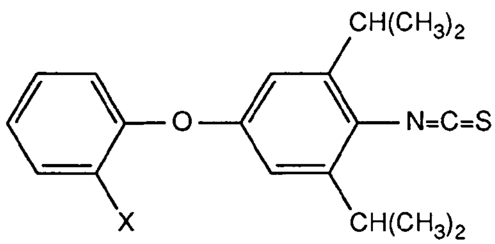

(II)

worin X die unter Formel I gegebene Bedeutung hat, mit tert.Butylamin der Formel III

$H_2N\text{-}C(CH_3)_3$ (III)

zum Thiohamstoff der Formel Ia

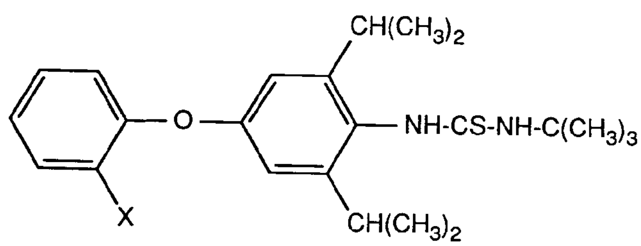

(Ia)

umsetzt und gewünschtenfalls
b) den erhaltenen Thioharnstoff der Formel Ia durch Abspalten von Schwefelwasserstoff in das Carbodiimid der Formel Ib

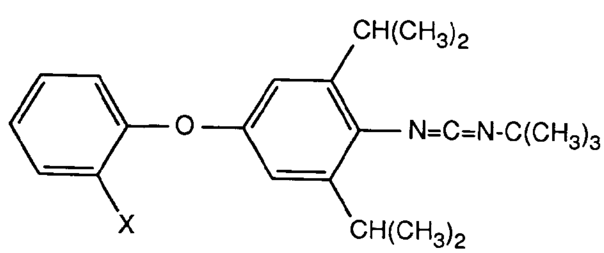

(Ib)

überführt.

6. Schädlingsbekämpfungsmittel, welches als aktive Komponente mindestens ein 3,5-Diisopropyl-diphenyläther-Derivat der Formel I gemäss Anspruch 1 enthält.

7. Mittel gemäss Anspruch 6, dadurch gekennzeichnet, dass es neben dem Wirkstoff der Formel I noch mindestens einen Trägerstroff enthält.

8. Verfahren zur Bekämpfung von tier- und pflanzenschädigenden Insekten und Vertretern der Ordnung Akarina, dadurch gekennzeichnet, dass man die Schädlinge oder ihren Lebensraum mit einer wirksamen Menge eines 3,5-Diisopropyl-diphenyläther-Derivats der Formel I gemäss Anspruch 1 behandelt.

9. Verwendung eines 3,5-Diisopropyl-diphenyläther-Derivats der Formel I gemäss Anspruch 1 zur Bekämpfung von schädlichen Insekten und Vertretern der Ordnung Akarina.

10. Verwendung gemäss Anspruch 9, dadurch gekennzeichnet, dass es sich bei den Schädlingen um pflanzenschädigende Insekten handelt.

11. Verwendung gemäss Anspruch 10 gegen pflanzenschädigende Akarina.

**Patentansprüche für folgenden Vertragsstaat : ES**

1. Verfahren zur Herstellung der 3,5-Diisopropyl-diphenyläther-Derivate der Formel I

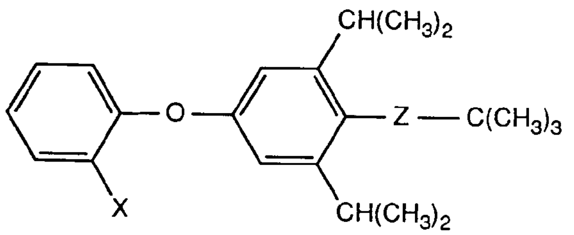

(I)

worin X Fluor oder Chlor und
Z die Brücke -NH-CS-NH- oder -N=C=N- bedeuten, dadurch gekennzeichnet, dass man
a) ein Isothiocyanat der Formel II

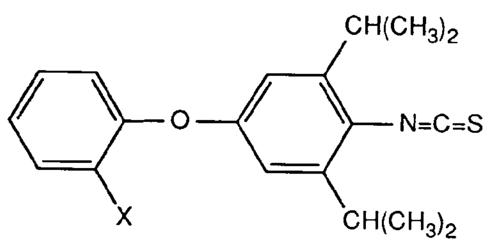

(II)

worin X die unter Formel I gegebene Bedeutung hat, mit tert.Butylamin der Formel III

$H_2N\text{-}C(CH_3)_3$ (III)

zum Thioharnstoff der Formel Ia

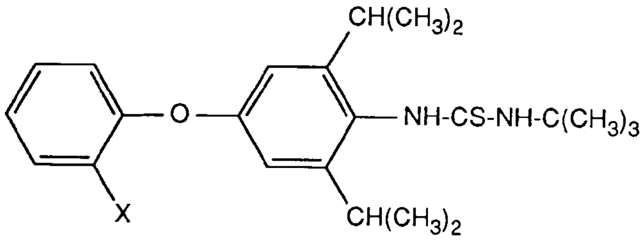

(Ia)

umsetzt und gewünschtenfalls

b) den erhaltenen Thioharnstoff der Formel Ia durch Abspalten von Schwefelwasserstoff in das Carbodiimid der Formel Ib

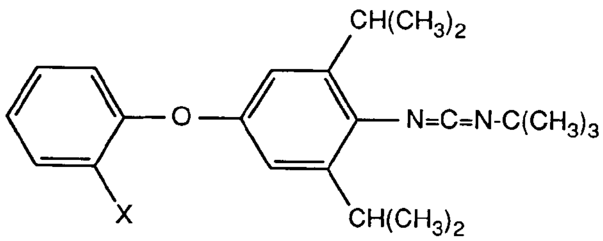

(Ib)

überführt.

2. Verfahren gemäss Anspruch 1, dadurch gekennzeichnet, dass X für Chlor steht.

3. Verfahren gemäss Anspruch 1 zur Herstellung von N-2,6-Diisopropyl-4-(2-chlorphenoxy)-phenyl-N'-tert.butyl-thioharnstoff.

4. Verfahren gemäss Anspruch 1 zur Herstellung von N-2,6-Diisopropyl-4-(2-chlorphenoxy)-phenyl-N'-tert.butyl-carbodiimid.

5. Schädlingsbekämpfungsmittel, welches als aktive Komponente mindestens ein 3,5-Diisopropyl-diphenyläther-Derivat der Formel I gemäss Anspruch 1 enthält.

6. Mittel gemäss Anspruch 5, dadurch gekennzeichnet, dass es neben dem Wirkstoff der Formel I noch mindestens einen Trägerstroff enthält.

7. Verfahren zur Bekämpfung von tier- und pflanzenschädigenden Insekten und Vertretern der Ordnung Akarina, dadurch gekennzeichnet, dass man die Schädlinge oder ihren Lebensraum mit einer wirksamen Menge eines 3,5-Diisopropyl-diphenyläther-Derivats der Formel I gemäss Anspruch 1 behandelt.

8. Verwendung eines 3,5-Diisopropyl-diphenyläther-Derivats der Formel I gemäss Anspruch 1 zur Bekämpfung von schädlichen Insekten und Vertretern der Ordnung Akarina.

9. Verwendung gemäss Anspruch 8, dadurch gekennzeichnet, dass es sich bei den Schädlingen um pflanzenschädigende Insekten handelt.

10. Verwendung gemäss Anspruch 9 gegen pflanzenschädigende Akarina.

Europäisches Patentamt

# EUROPÄISCHER RECHERCHENBERICHT

Nummer der Anmeldung

EP 91 81 0787

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 175 649 (CIBA-GEIGY)<br>* Anspruch 1 *<br>--- | 1-11 | C 07 C 335/18<br>C 07 C 267/00<br>A 01 N 47/30<br>A 01 N 47/40 |
| D,A | GB-A-2 060 626 (CIBA-GEIGY)<br>* Seite 1, Formel (II) *<br>--- | 1-11 | |
| A | WO-A-8 203 390 (CIBA-GEIGY)<br>* Seite 3, Formel (II) *<br>--- | 1-11 | |
| A | EP-A-0 338 988 (CIBA-GEIGY)<br>* Anspruch 5; Formel 2 *<br>----- | 1-11 | |
| | | | **RECHERCHIERTE SACHGEBIETE (Int. Cl.5)**<br>C 07 C 335/00<br>C 07 C 267/00 |

Der vorliegende Recherchenbericht wurde für alle Patentansprüche erstellt

| Recherchenort | Abschlußdatum der Recherche | Prüfer |
|---|---|---|
| DEN HAAG | 13-01-1992 | ZAROKOSTAS K. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)